# EUROPEAN PATENT APPLICATION

(11) **EP 3 502 237 A1**
(43) Date of publication of application: **26.06.2019**
(21) Application number: 17799555.2
(22) Date of filing: 23.03.2017
(51) Int. Cl.: C12N 5/0775, A61K 31/661, C07F 9/09, C07F 9/655

(54) **COMPOSITION FOR PROMOTING GROWTH OF STEM CELLS COMPRISING PHYTOSPHINGOSINE-1-PHOSPHATE OR DERIVATIVES THEREOF, AND COMPOSITION FOR CULTURING MEDIA OF STEM CELLS COMPRISING SAME**

(30) Priority: 17.05.2016 KR 20160060177
(71) Applicant: Phytos Co., Ltd., Anyang-si, Gyeonggi-do 14056 (KR)
(72) Inventor: CHOI, Myeong Jun, Seoul 06286 (KR)
(74) Representative: V.O.
(86) International application number: PCT/KR2017/003091
(87) International publication number: WO 2017/200197

(57) **Abstract**

The present application relates to a composition for promoting proliferation of adult stem cells further comprising phytosphingosine-1-phosphate (P1P), O-cyclic P1P (cP1P), N-acetylphytosphingosine-1-phosphate (NAPS-1-P), and a pharmaceutically acceptable salt thereof in a basal medium, a composition for culturing adult stem cells comprising the same, or a composition for a serum-free or low-serum culture medium of adult stem cells. When the stem cells are cultured using the composition of the present application, the proliferation promotion, activity and differentiation capacity of the stem cells can be improved, and the death of the stem cells against external stress can be prevented.

## Description

### [TECHNICAL FIELD]

The present disclosure relates to a composition for stem cell culture medium. Specifically, the present disclosure relates to a composition for culturing adult stem cells using phytosphingosine-1-phosphate or derivatives thereof.

### [BACKGROUND ART]

Stem cells are multipotent cells that have the ability to produce various types of cells from a single cell, and collectively refer to cells that have the ability to regenerate cells in damaged areas of our body. Stem cells have a self-regenerative ability to continuously produce the same cells as themselves, have a differentiation capacity to differentiate into specific cells in specific environments, have an immune-regulatory capacity to regulate immune responses by reacting with immune cells, and also have an effect of promoting tissue regeneration and healing by production of cytokines, *etc.* Thus, they are being developed as therapeutic agents by many companies worldwide due to high applicability.

Stem cells can be divided into pluripotent stem cells that have the capacity to differentiate into all about 200 different cells constituting the human body according to the areas of the cells to be differentiated, and tissue-specific stem cells that are specialized so as to differentiate into specific types of cells. Further, according to the source from which stem cells are obtained, stem cells can be classified into embryonic stem cells obtained from embryos or blastocytes derived from fertilized eggs, and adult stem cells obtained from each body tissue of newborns after development or adults. Embryonic stem cells have the advantages of having excellent differentiation capacity, long telomeres, which make them young cells, and excellent stem cell functions. However, they have ethical problems, are difficult to culture, increase the risk of cancer, and are difficult to obtain in large quantities. Meanwhile, adult stem cells can be obtained in large quantities, but have a relatively low differentiation capacity. In addition, the adult stem cells do not carry a risk of carcinogenesis, thus are safe to be applied medically. As a result, all commercialized stem cell therapeutic agents are manufactured using adult stem cells. The commercialized stem cell therapeutic agents include Hearticellgram-AMI manufactured by Pharmicell, Cupistem manufactured by Anterogen, Neuronata-R manufactured by Corestem, Caristem manufacured by Medipost, etc. Adult stem cells are known to have no immune rejection during autologous transplantation and almost no immune rejection during allotransplantation. Further, even when injected in an undifferentiated state, they do not cause cancer and have a homing-effect, whereby cells migrate to a wound site during transplantation, and after migration, they secrete various physiologically active substances including growth factors, and have tissue-specific differentiation capacity whereby cells differentiate according to the characteristics of the surrounding tissues, thereby improving the environment of damaged tissues and promoting regeneration.

Among the adult stem cells, mesenchymal stem cells have an excellent proliferative capacity compared to somatic cells, and are pluripotent stem cells that can differentiate into bones, cartilages, and fats. They are much more genetically stabilized than embryonic stem cells and induced pluripotent stem cells, and have a low risk of carcinogenesis, and thus is being developed as a cell therapeutic agent for cartilage regeneration, and for the treatments of myocardial infarction, graft-versus-host disease, etc.

However, there are factors that have an effect when culturing the cells used for cell therapeutic agents, which include nutrients, pH, temperature, osmotic pressure, oxidative stress, etc. Further, a technique of controlling the concentration of some nutrients such as amino acids, inorganic salts and vitamins in a medium, a technique of using a natural drug extract, a technique of using growth factors, such as insulin, hydrocortisone, EGF and LIF, etc. have been developed so as to improve the proliferative capacity of undifferentiated mesenchymal stem cells, thereby contributing to the improvement of *in vitro* proliferation. However, there is still a disadvantage in that it is difficult to prevent premature aging of cells due to accumulation of active oxygen, so that it may be difficult to obtain the cells in large quantities.

In addition, in order to culture large quantities of stem cells used for cell therapeutic agents *in vitro,* a serum containing various components necessary for cell proliferation is used. The serum is widely used for cell survival and proliferation. However, the serum is a mixture among which only some components have been identified, and thus, the physiological activity of the serum in cell culture has not been completely understood. Normal fetal bovine serum is the most commonly used. The fetal bovine serum contains proteins, such as albumin, nutrients, hormones and adhesins that are necessary for cell culture. Further, it contains growth factors such as insulin and EGF, and also serves to neutralize toxic components of the medium. However, since the fetal bovine serum is drawn from a bovine fetus, there may be a significant difference in quality depending on the equipment and the level of technology of the supplier. Further, the components of the serum may not be the same depending on the production period, and thus it is necessary to use the serum having the same lot number, that is, the serum produced at the same time in the series of experiments or in the production of biotechnology products. In addition, it is difficult to analyze a trace amount of components contained in the fetal bovine serum, so that it is difficult to establish a reproducible test and production process, and since it is drawn from animals, it has a risk of contamination from other factors such as animal diseases, such as mad cow disease, or viruses. A serum-free medium currently being developed by companies is mostly a culture medium for isolating and purifying physiologically active substances produced by cells for industrialization, and a medium for stem cells are being developed based on the embryonic and induced pluripotent stem cell culture medium. In addition, a chemically defined medium is preferred for supply and cost stability, but a medium which can completely replace the serum has not been developed or is not generally available because it is preserved as the company's know-how.

Currently, development of a medium capable of rapid and large-scale culture of stem cells is the core technology of cell therapeutic agents, and various media are currently under development by leading companies. In case of foreign countries, serum-free media have been developed by Gibco (StemPro), Lonza (MSCGM), Stem cell technology (MesenCult XF), Promocell (MSCFM-DXF), CellGenix (CellGro), DS Pharma Biomedical (STK series), and are available on the market. In Korea, studies are actively conducted to improve stem cell proliferation and stem cell functions.

Korean Patent No. 10-1249801 relates to a composition for culturing stem cells including a melatonin receptor agonist in a basal medium and a method for culturing stem cells, but it relates to maintaining the stem cell functions or promoting stem cell differentiation.

Korean Patent Laid-open Publication No. 10-2013-0119683 relates to a stem cell culture medium which can replace a conventional stem cell culture medium containing fetal bovine serum, and discloses a stem cell culture medium containing a basal medium and a knockout serum replacement, and a method for culturing stem cells using the same.

Korean Patent Laid-open Publication No. 10-2015-0087047 relates to a serum-free medium composition for culturing stem cells and a method for culturing stem cells, in which a serum-free medium containing glabridin is disclosed.

In order to develop a cell therapeutic agent, it is necessary to develop a substance that increases the proliferation and activity of stem cells. Further, since the characteristics of the cultured stem cells are very different according to culture conditions, it is necessary to establish optimized culture conditions by comparing and analyzing various culture conditions.

### [DETAILED DESCRIPTION OF THE INVENTION]

### [Technical Problem]

It is one object of the present disclosure to provide a composition for promoting growth of stem cells including phytosphingosine-1-phosphate or a derivative thereof, and a composition for stem cell culture medium including the same.

### [Technical Solution]

One aspect of the present disclosure provides a composition for promoting proliferation of adult stem cells including P1P (phytosphingosine-1-phosphate), cP1P (O-cyclic P1P), and/or NAPS-1-P (N-acetyl phytosphingosine-1-phosphate), or a pharmaceutically acceptable salt thereof, a method for promoting proliferation of stem cells using the composition, or a method for culturing stem cells.

In one embodiment, the P1P (phytosphingosine-1-phosphate), cP1P (O-cyclic P1P), or NAPS-1-P (N-acetyl phytosphingosine-1-phosphate) which can be included in the composition according to the present disclosure may be contained at a concentration of about 1 nM to 50 µM. The optimum effect may be exhibited at the concentration range above, which is not limited thereto.

Another aspect of the present disclosure provides a composition for culturing adult stem cells including the aforementioned composition according to the present disclosure.

The composition for culturing adult stem cells according to the present disclosure may be used as a serum-free medium composition or a low-serum medium composition containing about 0.1 to 3% by weight of serum components.

Still another aspect of the present disclosure provides a kit for inhibiting cell death of adult stem cells *in vitro* including P1P (phytosphingosine-1-phosphate), cP1P (O-cyclic P1P), and/or NAPS-1-P (N-acetyl phytosphingosine-1-phosphate), or a derivate or a salt thereof, a method for inhibiting cell death of adult stem cells, including treating adult stem cells with the aforementioned compound, or a method for culturing adult stem cells.

Still further another aspect of the present disclosure provides a kit for promoting osteogenic differentiation of adult stem cells *in vitro* including P1P (phytosphingosine-1-phosphate), cP1P (O-cyclic P1P), and/or NAPS-1-P (N-acetyl phytosphingosine-1-phosphate), or a derivate or a salt thereof, or a method for promoting osteogenic differentiation of adult stem cells *in vitro,* including treating adult stem cells with the aforementioned compound.

Specifically, the present disclosure provides a composition for promoting proliferation of adult stem cells including P1P (phytosphingosine-1-phosphate), cP1P (O-cyclic P1P), and/or NAPS-1-P (N-acetyl phytosphingosine-1-phosphate), or a derivate or an acceptable salt thereof.

Further, according to the present disclosure, P1P (phytosphingosine-1-phosphate), cP1P (O-cyclic P1P), or NAPS-1-P (N-acetyl phytosphingosine-1-phosphate) may be contained at a concentration of about 1 nM to 50µM.

Further, the present disclosure provides a composition for culturing stem cells including the composition, or P1P (phytosphingosine-1-phosphate), cP1P (O-cyclic P1P), and/or NAPS-1-P (N-acetyl phytosphingosine-1-phosphate), or a derivative or an acceptable salt thereof.

The composition according to the present disclosure may, in particular, be serum free or contain about 0.1 to 3% by weight of serum components, and thus enables an effective culture of stem cells.

Further, the present disclosure provides a method for culturing adult stem cells, including culturing by treating adult stem cells with any one of the compositions disclosed herein, or P1P (phytosphingosine-1-phosphate), cP1P (O-cyclic P1P), and/or NAPS-1-P (N-acetyl phytosphingosine-1-phosphate), or a derivative or an acceptable salt thereof.

Further, the present disclosure provides a method for promoting proliferation of adult stem cells, including treating adult stem cells with any one of the compositions disclosed herein, or P1P (phytosphingosine-1-phosphate), cP1P (O-cyclic P1P), and/or NAPS-1-P (N-acetyl phytosphingosine-1-phosphate), or a derivative or an acceptable salt thereof.

Further, the present disclosure provides a method for inhibiting cell death of adult stem cells, including treating adult stem cells with any one of the compositions disclosed herein, or P1P (phytosphingosine-1-phosphate), cP1P (O-cyclic P1P), and/or NAPS-1-P (N-acetyl phytosphingosine-1-phosphate), or a derivative or an acceptable salt thereof.

Further, the present disclosure provides a method for promoting osteogenic differentiation of adult stem cells, including treating adult stem cells with any one of the compositions disclosed herein, or P1P (phytosphingosine-1-phosphate), cP1P (O-cyclic P1P), and/or NAPS-1-P (N-acetyl phytosphingosine-1-phosphate), or a derivative or an acceptable salt thereof.

In the method disclosed herein, the P1P (phytosphingosine-1-phosphate), cP1P (O-cyclic P1P), or NAPS-1-P (N-acetyl phytosphingosine-1-phosphate) may be contained at a concentration of about 1 nM to 50 µM, but is not limited thereto.

Further, the present disclosure provides use of P1P (phytosphingosine-1-phosphate), cP1P (O-cyclic P1P), and/or NAPS-1-P (N-acetyl phytosphingosine-1-phosphate), or a derivative thereof for promoting proliferation of adult stem cells.

Further, the present disclosure provides use of P1P (phytosphingosine-1-phosphate), cP1P (O-cyclic P1P), and/or NAPS-1-P (N-acetyl phytosphingosine-1-phosphate) or a derivative thereof for inhibiting cell death of adult stem cells.

Further, the present disclosure provides use of P1P (phytosphingosine-1-phosphate), cP1P (O-cyclic P1P), and/or NAPS-1-P (N-acetyl phytosphingosine-1-phosphate), or a derivative thereof for promoting osteogenic differentiation of adult stem cells.

Further, the present disclosure provides a kit for promoting proliferation of adult stem cells *in vitro* including P1P (phytosphingosine-1-phosphate), cP1P (O-cyclic P1P), and/or NAPS-1-P (N-acetyl phytosphingosine-1-phosphate), or a pharmaceutically acceptable salt thereof.

Further, the present disclosure provides a kit for inhibiting cell death of adult stem cells *in vitro* including P1P (phytosphingosine-1-phosphate), cP1P (O-cyclic P1P), and/or NAPS-1-P (N-acetyl phytosphingosine-1-phosphate), or a derivative or an acceptable salt thereof.

Further, the present disclosure provides a kit for promoting osteogenic differentiation of adult stem cells *in vitro* including P1P (phytosphingosine-1-phosphate), cP1P (O-cyclic P1P), and/or NAPS-1-P (N-acetyl phytosphingosine-1-phosphate), or a derivative or an acceptable salt thereof.

Further, the present disclosure provides a kit for culturing adult stem cells *in vitro* including P1P (phytosphingosine-1-phosphate), cP1P (O-cyclic P1P), and/or NAPS-1-P (N-acetyl phytosphingosine-1-phosphate), or a derivative or an acceptable salt thereof.

### [ADVANTAGEOUS EFFECTS]

The present disclosure relates to a composition for promoting growth of stem cells, including one or more compounds selected from the group consisting of P1P (phytosphingosine-1-phosphate), cP1P (O-cyclic P1P), NAPS-1-P (N-acetyl phytosphingosine-1-phosphate), and pharmaceutically acceptable salts thereof, a composition for stem cell culture medium including the same, or a composition for serum-free or low-serum culture medium of stem cells. When stem cells are cultured using the composition of the present disclosure, the proliferation of stem cells can be promoted, the activity and differentiation capacity of stem cells can be improved, and cell death of stem cells against external stress can be prevented, and thus the composition of the present disclosure can be effectively used for culturing stem cells.

In addition, the stem cell culture medium according to the present disclosure has a remarkably high viability of stem cells in a serum-free or 2% serum medium, in which the serum components were reduced, as compared with a basal medium, and thus can be effectively used for the development of a serum-free medium and a low serum medium.

### [BRIEF DESCRIPTION OF DRAWINGS]

FIG. 1 illustrates the efficacies of P1P (phytosphingosine-1-phosphate), cP1P (O-cyclic P1P), and NAPS-1-P (N-acetyl phytosphingosine-1-phosphate) on the proliferation of mesenchymal stem cells, according to one embodiment of the present disclosure.
FIG. 2 illustrates the effects due to the concentrations of P1P, cP1P, and NAPS-1-P on the proliferation of human-derived adipose stem cells, according to one embodiment of the present disclosure.
FIG. 3a shows the effect due to the concentration of P1P on colony formation of human-derived adipose stem cells, according to one embodiment of the present disclosure.
FIG. 3b are optical microscopic images in which the changes in colony are observed in terms of size and number according to the concentration of P1P, according to one embodiment of the present disclosure.
FIG. 4 illustrates the effect of irradiation of radioactive rays on the proliferation of mesenchymal stem cells.
FIG. 5 illustrates the inhibitory effect of P1P on cell death of mesenchymal stem cells induced by radioactive rays.
Fig. 6 illustrates the inhibitory effect of P1P on cell death of human-derived adipose stem cells induced by oxidative stress.
FIG. 7 illustrates the effect of P1P and cP1P on osteogenic differentiation of human-derived adipose stem cells.
FIG. 8a illustrates the effect of P1P, cP1P, and NAPS-1-P on the proliferation of adipose stem cells in serum-free medium.
FIG. 8b illustrates the effect of P1P, cP1P, and NAPS-1-P on the proliferation of adipose stem cells in 2% low-serum medium.

### [DETAILED DESCRIPTION OF THE EMBODIMENTS]

The present disclosure is based on the findings that phytosphingosine-1-phosphate or a derivative thereof promotes stem cell proliferation and that it shows an excellent proliferation effect in a serum-free or low-serum medium.

Accordingly, in one aspect of the present disclosure, there is provided a composition for promoting growth or proliferation of stem cells, including one or more compounds selected from the group consisting of P1P (phytosphingosine-1-phosphate), cP1P (O-cyclic P1P), NAPS-1-P (N-acetyl phytosphingosine-1-phosphate), and a pharmaceutically acceptable salt thereof, or a composition for stem cell culture medium including the same.

The P1P (phytosphingosine-1-phosphate, represented by Chemical Formula I below), cP1P (O-cyclic P1P, represented by Chemical Formula II below), and NAPS-1-P (N-acetyl phytosphingosine-1-phosphate, represented by Chemical Formula III below) are derivatives of sphingosine-1-phosphate (S1P) and substances that have been developed by the present inventors for various purposes and have been granted patents. They are disclosed in Korean Patent Nos. 10-1003532 and 10-1340556 (Novel substance and use thereof for treating hair loss), and 10-1514970 (Composition for treatment and prevention of atopic dermatitis or skin wounds) and are represented by the following Chemical Formulas I, II and III, respectively.

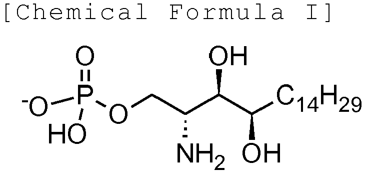

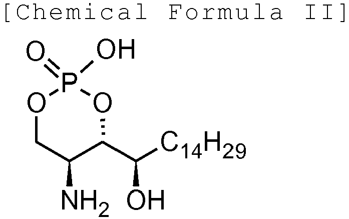

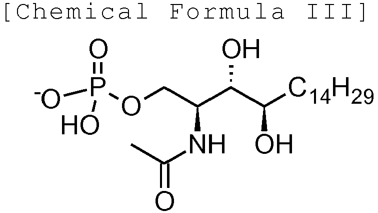

The compounds according to the present disclosure may be prepared using common knowledge known in the field of organic chemistry, for example, they may be prepared by the method disclosed in S. Li, W.K. Wilson, G.J. Schroepfer, Chemical synthesis of D-ribo-phytosphingosine-1-phosphate, potential modulator of cellular processes. J. Lipid Res. 40: 117-125, 1999, or by the method disclosed in Korean Patent No. 10-1514970.

The pharmaceutically acceptable salts of the compound of Chemical Formula I, II or III, or solvates thereof may be suitably prepared or selected using knowledge known to those skilled in the art in the field of organic chemistry.

As used herein, the salt refers to a salt that is physiologically acceptable and usually does not invoke common allergic or similar reactions when administered to humans. Preferably, the salt may be an acid addition salt formed from a free acid. As the free acid, an organic acid or inorganic acid may be used. The organic acid includes, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid and aspartic acid. Further, the inorganic acid includes, but is not limited to, hydrochloric acid, bromic acid, sulfuric acid, and phosphoric acid. In one embodiment according to the present disclosure, the pharmaceutically acceptable salt may be present as an acid addition salt in which the compound of the Chemical Formula I, II or III forms a salt with a free acid. In addition, the compound of the Chemical Formula I, II or III according to the present disclosure may include not only pharmaceutically acceptable salts, but also all salts, hydrates and solvates that can be prepared by a conventional method. The compound of the Chemical Formula I, II or III may be stabilized by an anion that can be paired with an ammonium cation in the compound, and the anion may be any anion which is pharmaceutically acceptable and can be paired with an ammonium cation, and for example, the anion may be iodide (I⁻), sulfonate (SO₃²⁻), chloride (Cl⁻), *etc.*, but is not limited thereto.

As used herein, the stem cells are multipotent cells that have the ability to produce various types of cells from a single cell, and collectively refer to cells that have the capacity to regenerate cells in damaged areas of our body. The stem cells have a self-regenerative capacity to continuously produce the same cells as themselves, have a differentiation capacity to differentiate into specific cells in specific environments, and have an immune-regulatory capacity to regulate immune responses by reacting with immune cells.

The stem cells can be divided into pluripotent stem cells that have the capacity to differentiate into all about 200 different cells constituting the human body according to the area of the cells to be differentiated, and tissue-specific stem cells that are specialized to differentiate into specific types of cells. Further, according to the source from which the stem cells are obtained, the stem cells can be classified into embryonic stem cells obtained from embryos or blastocytes derived from fertilized eggs, and adult stem cells obtained from each body tissue of newborns after development or adults.

In one embodiment according to the present disclosure, in particular, the adult stem cells, for example, human-derived mesenchymal stem cells or adipose stem cells, are included herein.

P1P, or a derivative compound or a salt thereof may be used for a composition or a medium composition for culturing cells including the aforementioned substance for the purpose of promoting proliferation or growth of stem cells or achieving the effects described herein, by adding to a conventional medium used for culturing stem cells.

It has been confirmed that the addition of P1P or a derivative or a salt thereof to a medium promotes the proliferation of stem cells and improves the differentiation capacity, and that it functions in preventing cell death of stem cells induced by radioactive rays and oxidative stress.

As the medium, any medium widely known to those skilled in the art to which the present disclosure belongs may be used without limitation. The medium may be artificially synthesized, and a commercially prepared medium may be used. Examples of the commercially prepared medium may include DMEM (Dulbecco's Modified Eagle's Medium), MEM (Minimal Essential Medium), BME (Basal Medium Eagle), RPMI 1640, F-10, F-12, α-MEM (α-Minimal essential Medium), G-MEM (Glasgow's Minimal Essential Medium), IMDM (Isocove's Modified Dulbecco's Medium), and MEF, but are not limited thereto.

In one embodiment, the medium may be DMEM. DMEM (Dulbecco's modified Eagle's medium) may be used, but not limited thereto.

The P1P or a derivative compound thereof may be contained at an appropriate concentration depending on the type of specific cells as intended as long as it complies with the purpose of the present disclosure. In one embodiment, it may be contained at a concentration of 0.1 nM to 100 µM, preferably 1 nM to 50 µM. In another embodiment, the compound may be contained at a concentration of 10 nM to 1 µM.

Meanwhile, when P1P or a derivative thereof according to the present disclosure was added to the medium, it was confirmed that the stem cell proliferation was carried out even in the serum-free medium or in the low-serum medium in which the serum components were reduced.

Accordingly, in another aspect, the present disclosure relates to a composition for culturing stem cells in a serum-free or low-serum medium, including one or more compounds selected from the group consisting of P1P (phytosphingosine-1-phosphate), cP1P (O-cyclic P1P), NAPS-1-P (N-acetyl phytosphingosine-1-phosphate), and a pharmaceutically acceptable salt thereof.

In one embodiment, the P1P or a derivative compound thereof may be contained at an appropriate concentration depending on the type of specific cells as intended as long as it complies with the purpose of the present disclosure. In one embodiment, it may be contained at a concentration of 0.1 nM to 100 µM, preferably 1 nM to 50 µM. In another embodiment, the compound may be contained at a concentration of 10 nM to 1 µM.

As used herein, the serum-free medium refers to any culture medium containing no more than a certain amount of serum derived from an animal including human (animal-derived serum). For example, the serum-free medium may contain less than 0.1% or less than 0.01% by weight of an animal-derived serum relative to the total weight of the composition, and may specifically contain no animal-derived serum. The present disclosure provides a serum-free medium composition including the compound of I, II or III instead of an animal-derived serum necessary for proliferation and culture of stem cells. Therefore, in the present disclosure, it is possible to stably proliferate and culture stem cells to such an extent that the animal-derived serum can be replaced, and to establish a reproducible test and production processes.

In the low-serum medium, 0.1 to 3% by weight of fetal bovine serum (FBS), which is a commonly used serum for cell culture, is added, and a substance containing components similar to an animal-derived serum, for example, a bovine pituitary extract may be used. In one embodiment, the low serum medium is added with 2% by weight of fetal bovine serum.

Also, the present inventors have found that, when stem cells are cultured using a medium containing the P1P and a derivative compound thereof, the osteogenic differentiation of human-derived adipose stem cells is promoted in an osteogenic differentiation medium.

Accordingly, in still another aspect, the present disclosure relates to a composition for promoting osteogenic differentiation of stem cells, including one or more compounds selected from the group consisting of P1P (phytosphingosine-1-phosphate), cP1P (O-cyclic P1P), NAPS-1-P (N-acetyl phytosphingosine-1-phosphate), and a pharmaceutically acceptable salt thereof.

As used herein, the 'stem cells' are the same as described above, and may be preferably derived from various adult tissues such as bone marrows, adipose tissues, cord blood, peripheral blood, neonatal tissues, and placenta, and bone marrow-derived cells, but are not limited thereto.

As used herein, the 'osteogenic differentiation' refers to differentiation into osteoblasts constituting the human bone.

The bones of the human body are gradually degraded every day and filled with new bones by the amount of the degraded bones, so that the homeostasis is maintained. There are osteoclasts, which degrade bones, and osteoblasts, which regenerate bones, and when the activity of one type of the osteoclasts and osteoblasts is increased or decreased, several diseases may be caused due to the destruction of homeostasis. The increase in the activity of the osteoclasts that absorb bones causes diseases, such as osteoporosis, in which the degradation of bones is promoted and the bones become thin and are easily broken, and the increase in the activity of the osteoblasts causes bone deformities or bone calcification due to increased bone density.

The composition of the present disclosure may be used for the prevention and treatment of bone diseases. As used herein, the 'bone disease' refers to a disease that causes a disorder of bone tissue or destruction of bone tissue due to imbalance of osteoblasts and osteoclasts. The bone-related disease is not particularly limited as long as the symptoms can be prevented, improved, alleviated or treated by the composition provided by the present disclosure, and may include growth retardation, bone fracture, rickets, osteomalacia, scoliosis, pelvic deformity, osteoporosis, rheumatoid arthritis, periodontal disease, hyperparathyroidism, Paget's disease, metastatic bone cancers, etc.

As use herein, the "prevention or treatment of bone disease" refers to any activity that prevents the occurence of the bone-related diseases, or alleviates or positively changes symptoms of the diseases, and include prevention, reduction, improvement in the symptoms of the bone diseases, amelioration of the painful symptoms, reduction in the incidence of the bone diseases, or any other changes in the patient that increase the treatment results.

In still further another aspect, the present disclosure provides a kit for inhibiting cell death of adult stem cells *in vitro*, including P1P (phytosphingosine-1-phosphate), cP1P (O-cyclic P1P), or NAPS-1-P (N-acetyl phytosphingosine-1-phosphate), or a pharmaceutically acceptable salt thereof.

In still further another aspect, the present disclosure provides a kit for promoting proliferation of adult stem cells *in vitro,* including P1P (phytosphingosine-1-phosphate), cP1P (O-cyclic P1P), or NAPS-1-P (N-acetyl phytosphingosine-1-phosphate), or a pharmaceutically acceptable salt thereof.

In still further another aspect, the present disclosure provides a kit for promoting osteogenic differentiation of adult stem cells *in vitro,* including P1P (phytosphingosine-1-phosphate), cP1P (O-cyclic P1P), or NAPS-1-P (N-acetyl phytosphingosine-1-phosphate), or a pharmaceutically acceptable salt thereof.

The active ingredients contained in the kit according to the present disclosure may be referred to those mentioned above and may further include additional ingredients and methods for providing desired effects *in vitro.*

In still further another aspect, the present disclosure provides a method for proliferating stem cells, a method for culturing stem cells, a method for inhibiting cell death of stem cells, and a method for promoting osteogenic differentiation of stem cells, including treating stem cells with the composition according to the present disclosure. For more detailed steps and ingredients involved in the aforementioned methods, refer to those mentioned above and the descriptions of Examples shown below.

Hereinafter, Examples are provided to help understanding of the present disclosure. However, these Examples are given for illustrative purposes only to help understanding of the present disclosure, and the scope of the invention is not intended to be limited to or by these Examples.

### Examples

### Materials and Methods of Experiment

### Preparation of P1P and Derivatives Thereof

P1P (phytosphingosine-1-phosphate), cP1P (O-cyclic P1P), NAPS-1-P (N-acetyl phytosphingosine-1-phosphate) were prepared as described in Korean Patent No. 1514970.

### Measurement of Stem Cell Proliferation Capacity

Human mesenchymal stem cells were cultured in DMEM medium (Invitrogen) containing 10% fetal bovine serum and 1% penicillin/streptomycin at 37°C under 5% CO₂, and the medium was changed every 3 days. After culturing the cells for 24 hours, P1P, cyclic P1P, and NAPS-1-P were dissolved in DMSO (Sigma) to a concentration of 2 mM, and appropriately diluted and added to the medium such that the final concentration was 1 µM. The absorbance was measured at 450 nm using Ez-Cytox at 24 hours (1 day), 48 hours (2 days), 120 hours (5 days) and 168 hours (7 days) after the addition, and the proliferation capacity of the stem cells according to P1P, cyclic P1P and NAPS-1 were measured. For the control group, an experiment was performed under the same conditions as above except that P1P, cyclic P1P and NAPS-1-P were not included.

### Measurement of Colony-Forming Capacity using Adipose Stem Cells

Human-derived adipose stem cells were cultured in DMEM medium containing 10% fetal bovine serum and 1% penicillin/streptomycin at 37°C under 5% CO₂, and the medium was changed every 5 days. After culturing the cells for 24 hours, P1P was dissolved in DMSO to a concentration of 2 mM, and appropriately diluted and added to the medium such that the final concentrations were 10 nM, 100 nM, 1000 nM. When colonies were observed 14 to 21 days after culture, the cells were fixed and stained. First, the cells were washed twice with PBS solution, and 3 mL of 3.7% formaldehyde was loaded per 25 T flask, then the cells were allowed to stand at room temperature for 10 minutes to fix the stem cells. After fixation, the cells were washed with PBS, and 3 mL of crystal violet was added to the flask and allowed to stand at room temperature for 30 minutes to 1 hour to stain the stem cells. Thereafter, the cells were washed with tap water and dried at room temperature. The number of colonies was measured using a microscope, and cell proliferation and colony-forming capacity were compared. Further, the size of colonies was also observed using an optical microscope. For the control group, an experiment was performed under the same conditions as above except that P1P was not included.

### Comparison of Viability of Stem Cells Irradiated with Radioactive Rays

Human mesenchymal stem cells were cultured in DMEM medium containing 10% fetal bovine serum and 1% penicillin/streptomycin at 37°C under 5% CO₂, and the medium was changed every 3 days. After culturing the cells for 24 hours, the stem cells were irradiated with radioactive rays at a dose of 10 and 30 Gy for 6.77 min and 20.31 min, respectively, and 1 µM of P1P was added 6 hours before exposure to radioactive rays. The absorbance was measured at 450 nm using Ez-Cytox at 24 hours (1 day), 48 hours (2 days), 120 hours (5 days) and 168 hours (7 days) after irradiation of radioactive rays, and the effect of P1P on the viability of stem cells against the radioactive rays was measured. For the control group, an experiment was performed under the same conditions as above except that P1P was not included.

### Comparison of Viability of Stem Cells with Oxidative Stress Induced by Hydrogen Peroxide (H₂O₂)

Human-derived adipose stem cells were cultured in DMEM medium containing 10% fetal bovine serum and 1% penicillin/streptomycin at 37°C under 5% CO₂. After culturing the cells for 24 hours, P1P, cP1P and NAPS-1-P were dissolved in DMSO to a concentration of 2 mM, and appropriately diluted and added to the medium such that the final concentrations were 10 nM, 100 nM, 1000 nM. After 1 hour of culture, 350 µM of H₂O₂ was added to the stem cells and cultured. After 6 hours, the absorbance was measured at 450 nm using Ez-Cytox, and the effect of P1P and derivatives thereof on the viability of stem cells against oxidative stress was measured. For the control group, an experiment was performed under the same conditions as above except that P1P, cyclic P1P, and NAPS-1-P were not included.

### Comparison of Improvement of Osteogenic Differentiation Capacity of PIP and Derivatives Thereof

Human-derived adipose stem cells were cultured in DMEM medium containing 10% fetal bovine serum and 1% penicillin/streptomycin at 37°C under 5% CO₂. After 24 hours of culture, P1P and derivatives thereof were diluted with the medium and added. After 24 hours of culture, the medium was replaced with a differentiation medium and changed with a differentiation medium every 3 days. The cells were cultured in alpha-DEM medium containing 10% fetal bovine serum, 10 nM dexamethasone, 0.2 mM ascorbic acid, 10 mM beta-glycerol phosphate and 1% penicillin/streptomycin at 37°C under 5% CO₂ as a composition for the differentiation medium. After 2 to 4 weeks, the stem cells were fixed and stained with Alizarin Red S. After removing the stem cell culture medium, the cells were washed 3 times with PBS, and after removal of PBS, the cells were fixed using 4% formaldehyde solution for 20 minutes at room temperature. After washing the cells 3 times with PBS, the cells were treated with a 2% Alizarin Red S solution for 20 minutes. After removing the Alizarin Red S solution and washing the cells with PBS, the area where calcium deposition occurred was observed in red. For the control group, an experiment was performed under the same conditions as above except that P1P, cyclic P1P, and NAPS-1-P were not included.

### Comparison of Stem Cell Viability According to Concentration of Fetal Bovine Serum

Human-derived adipose stem cells were cultured in DMEM medium having different concentrations of fetal bovine serum at 37°C under 5% CO₂. After culturing for 24 hours, the cells were washed twice with PBS at 37°C to remove the serum components. Then, the cells were cultured in new DMEM medium containing 1 µM P1P and 1% fetal bovine serum. After 4 days, the absorbance was measured at 450 nm using an Ez-Cytox Cell Viability Assay Kit (Daeil Biotech) to measure the viability of stem cells according the concentration of fetal bovine serum and P1P. For the control group, an experiment was performed under the same conditions as above except that P1P was not included.

### Example 1. Confirmation of Effect of Promoting Stem Cell Proliferation Capacity by P1P and Derivatives Thereof

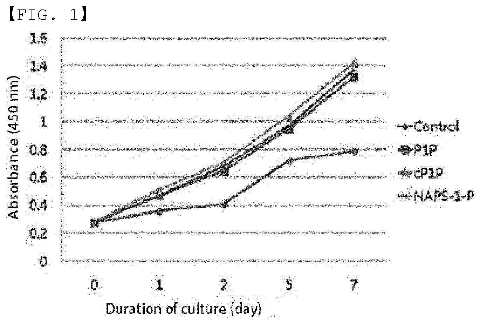

The growth promoting-capacity of P1P, cP1P, and NAPS-1-P on human mesenchymal stem cells was examined. As a result, three substances promoted the proliferation of stem cells to a similar level at a concentration of 1 uM. When compared according to the time, the increase in the proliferation rate was the highest after 2 days, and when cultured for 7 days with the addition of P1P and derivatives thereof, it can be seen that the proliferation effect was increased by 2-fold. When P1P was added to the medium, it can be seen that the growth of stem cells occurred very rapidly (FIG. 1).

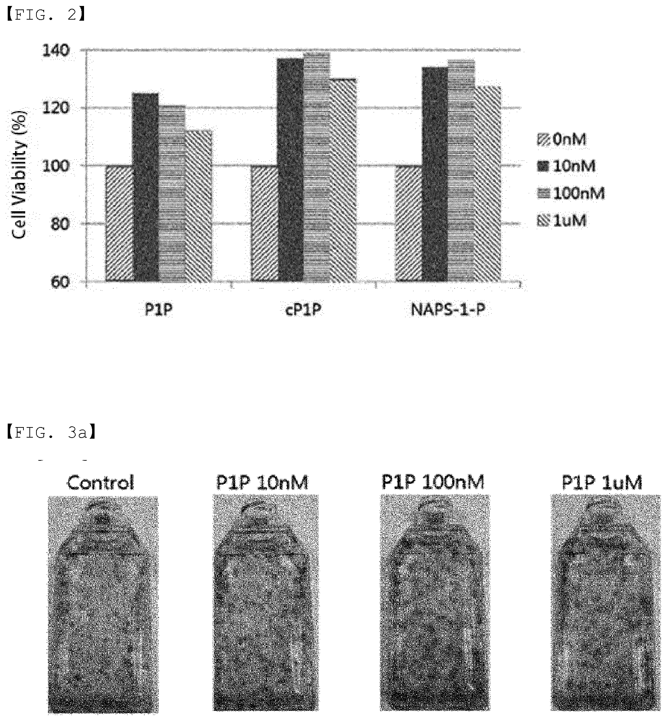

In addition, the growth promoting-capacity of human-derived adipose stem cells was examined. As a result, it was found that the P1P and the derivatives thereof promoted cell growth under the condition that the cells were cultured for 2 days according to different concentrations of P1P and derivatives. In particular, cP1P and NAPS-1-P showed a higher proliferation effect, and the optimal proliferation effect was observed at the concentrations of 10 nM and 100 nM (FIG. 2).

### Example 2. Effect of Promoting Colony-Forming Capacity of Stem Cells by P1P

The colony-forming capacity, which is a characteristic of human-derived adipose stem cells, was compared. As a result, it was confirmed that when P1P was added, the colony forming capacity was increased and the size of colony was also changed. It was also confirmed that as the concentration of P1P increased, the number and size of colonies also increased (FIG. 3a).

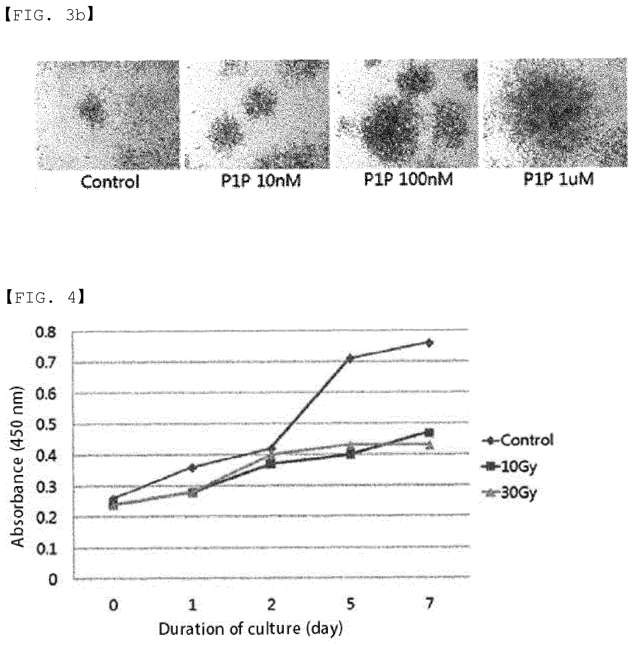

As a result of observation using an optical microscope after staining, the size of colony was very small in the absence of P1P, but when P1P was added to the medium, the size of colony was increased (FIG. 3b).

In general, when cells become active, their size tends to increase. Thus, the above results showed that P1P increased not only the number of stem cells, but also the activity thereof. Table 1 shows the distribution of the size of colony by P1P concentration. It was found that as the concentration of P1P increased, the number of particles with a large colony size increased significantly. That is, when P1P was not added, the ratio of the size of 0 to 2 mm was 58% and the particle size of 5 mm or more was 7%. However, when the P1P concentration was increased, the particle ratio of the size of colony of 0 to 2 mm was remarkably decreased, and the ratio of the size of colony of 5 mm or more tended to increase significantly. Thus, it can be seen that the colony-forming capacity of stem cells was promoted when P1P was added to the culture medium.

**[Table 1] Distribution of colony size according to P1P concentration**

| Size of colony | PIP concentration (nM) | | | |
|---|---|---|---|---|
| | 0 | 10 | 100 | 1000 |
| 0-2 mm | 58 | 49 | 43 | 34 |
| 2-5 mm | 35 | 38 | 34 | 39 |
| > 5 mm | 7 | 13 | 23 | 27 |

### Example 3. Inhibitory Effect of P1P on Cell Death of Stem Cells Induced by Radioactive Rays

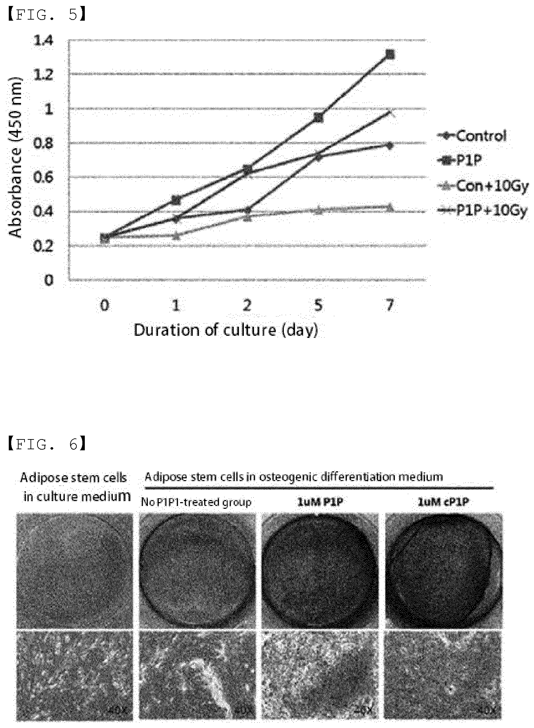

When radioactive rays were irradiated while culturing mesenchymal stem cells, cell death of stem cells occurred. When the stem cells were irradiated with ionizing radioactive rays at a dose of 10 to 30 Gy, the proliferation of stem cells was reduced and the process of cell death occurred. A similar degree of cell death was observed regardless of the amount of radioactive rays (FIG. 4). In this regard, in the experiment in which the inhibitory effect of cell death by P1P was observed, 10 Gy of radioactive rays were irradiated on the stem cells. It was confirmed that when the stem cells were not treated with P1P, cell proliferation was reduced due to cell death caused by irradiation of radioactive rays, whereas when P1P was added, cell death decreased and cell proliferation occurred. Therefore, these results indicate that P1P has an effect of inhibiting cell death of stem cells induced by external noxious stimuli (radioactive rays) (FIG. 5).

### Example 4. Inhibitory Effect of P1P on Cell Death of Stem Cells Induced by Oxidative Stress

Oxidative stress greatly influences cell viability when human-derived adipose stem cells are cultured in large quantities. In this regard, the effect of oxidative stress on stem cell proliferation was investigated, and as a result, it was confirmed that when 350 µM of hydrogen peroxide was added to the medium, the stem cells were rapidly killed, whereas when 100 nM of P1P was added, almost no cell death was observed and the stem cells survived. Table 2 shows the changes in viability according to the concentrations of P1P, cP1P, and NAPS-1-P. It can be seen that cell death of stem cells induced by oxidative stress was remarkably suppressed regardless of the type of the treated substance. Therefore, these results indicate that P1P and derivatives thereof have an effect of inhibiting cell death of stem cells induced by external stimuli (oxidative stress induced by hydrogen peroxide).

**[Table 2] Effect of P1P on the changes in viability by oxidative stress**

| Concentration | No H₂O₂-treated group | Cell viability (%) by H₂O₂ treatment (350 uM) | | | |
|---|---|---|---|---|---|
| | | Control | P1P | cP1P | NAPS-1-P |
| 0 nM | 100% | 10% | 10% | 10% | 10% |
| 10 nM | - | - | 71% | 77% | 75% |
| 100 nM | - | - | 81% | 84% | 83% |
| 1000 nM | - | - | 83% | 86% | 86% |

### Example 5. Effect of Stem Cell Culture in Serum-Free or Low-Serum Medium Containing P1P and Derivatives Thereof

Serum components are essential for the growth of normal cells as well as stem cells. However, when cells are cultured in large quantities, there are problems in that the serum components are very expensive and it is difficult to ensure reproducibility due to slight differences in the components in each production lot, and also, undesirable results can be obtained from unknown components in the serum. Thus, media using chemically defined components are actively being developed. Therefore, development of a low-serum or serum-free medium is required.

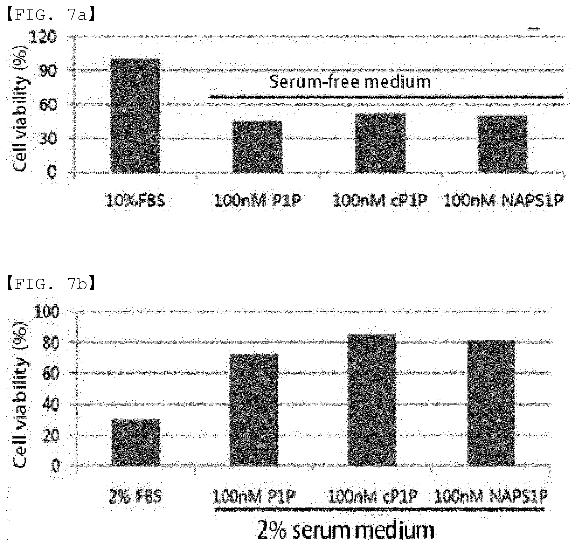

Accordingly, in the present disclosure, the effect of P1P on the proliferation of stem cells was observed by varying the concentrations of fetal bovine serum in DMEM medium. When 1 µM of P1P and derivatives thereof were added to the serum-free medium, stem cell proliferation occurred, but the viability was about 50% as compared with 10% fetal bovine serum (FIG. 7a). In addition, when the content of fetal bovine serum was reduced to 2%, the viability was found to be about 70 to 80% (FIG. 7b). These results indicate that P1P, cP1P, and NAPS-1-P can be effectively used in the development of serum-free and low-serum media.

### Example 6. Effect of P1P and Derivatives Thereof on Improvement of Osteogenic Differentiation of Adipose Stem Cells

In order to confirm whether human-derived adipose stem cells have the capacity to differentiate into various cells, the osteogenic differentiation capacity of the adipose stem cells was measured. Although the adipose stem cells proliferated well in a nutrient medium, when the medium was replaced with an osteogenic differentiation medium, the osteogenic differentiation capacity varied significantly depending on the presence or absence of P1P. The osteogenic differentiation occurred minimally in the osteogenic differentiation medium in the absence of P1P, but when P1P and cP1P were added, the osteogenic differentiation capacity was remarkably improved (FIG. 6). Therefore, it was confirmed that P1P and the derivatives thereof not only promoted the proliferation of stem cells but also improved the differentiation capacity.

Development of materials that enhance the proliferation and activity of stem cells is essential for the development of cell therapeutic agents using stem cells. In particular, the development of materials that can replace serum components is inevitable. P1P, cP1P, and NAPS-1-P not only have the effect of rapidly proliferating stem cells, increasing the activity of stem cells and improving the differentiation capacity, but also functions in protecting cell death of stem cells against external stimuli (radioactive rays and oxidative stress). Therefore, these materials are inevitably required for large-scale culture of stem cells for use in cell therapeutic agents and regenerative medicine. More importantly, when the above materials are included, stem cells can survive even in a serum-free medium without serum components essential for stem cell culture. Further, the viability was significantly high in the 2% serum medium in which the serum components were reduced. Thus, they can be effectively used for the development of a serum-free medium and a low-serum medium.

While the exemplary embodiments of the present disclosure have been described in detail, it is to be understood that the scope of the present disclosure is not limited to the disclosed embodiments, but, on the contrary, is intended to cover various modifications and equivalent arrangements made by those skilled in the art using the basic concepts of the present disclosure defined in the following appended claims.

All technical terms used herein have the same meanings that are understood by a skilled person in this art, unless otherwise specified. The contents of all publications disclosed in the present specification as a reference document are incorporated into the present disclosure.

## Claims

1. A composition for promoting proliferation of adult stem cells comprising P1P (phytosphingosine-1-phosphate), cP1P (O-cyclic P1P), and/or NAPS-1-P (N-acetyl phytosphingosine-1-phosphate), or a pharmaceutically acceptable salt thereof,

2. The composition according to claim 1, wherein the P1P (phytosphingosine-1-phosphate), cP1P (O-cyclic P1P), or NAPS-1-P (N-acetyl phytosphingosine-1-phosphate) is contained at a concentration of 1 nM to 50 µM.

3. A composition for culturing adult stem cells comprising the composition according to claim 1 to 2.

4. The composition for culturing adult stem cells according to claim 3, wherein the composition for culturing stem cells is serum free or contains 0.1 to 3% by weight of serum components.

5. A method for culturing adult stem cells, comprising culturing by treating adult stem cells with the composition according to claim 3 or 4.

6. A method for promoting proliferation of adult stem cells, comprising treating adult stem cells with P1P (phytosphingosine-1-phosphate), cP1P (O-cyclic P1P), and/or NAPS-1-P (N-acetyl phytosphingosine-1-phosphate), or a derivative or a salt thereof.

7. A method for inhibiting cell death of adult stem cells, comprising treating adult stem cells with P1P (phytosphingosine-1-phosphate), cP1P (O-cyclic P1P), and/or NAPS-1-P (N-acetyl phytosphingosine-1-phosphate), or a derivative or a salt thereof.

8. A method for promoting osteogenic differentiation of adult stem cells, comprising treating adult stem cells with P1P (phytosphingosine-1-phosphate), cP1P (O-cyclic P1P), and/or NAPS-1-P (N-acetyl phytosphingosine-1-phosphate), or a derivative or a salt thereof.

9. The method according to any one of claims 6 to 8, wherein the P1P (phytosphingosine-1-phosphate), cP1P (O-cyclic P1P), or NAPS-1-P (N-acetyl phytosphingosine-1-phosphate) is contained at a concentration of 1 nM to 50 µM.

10. Use of P1P (phytosphingosine-1-phosphate), cP1P (O-cyclic P1P), and/or NAPS-1-P (N-acetyl phytosphingosine-1-phosphate), or a derivative thereof for promoting proliferation of adult stem cells.

11. Use of P1P (phytosphingosine-1-phosphate), cP1P (O-cyclic P1P), and/or NAPS-1-P (N-acetyl phytosphingosine-1-phosphate) or a derivative thereof for inhibiting cell death of adult stem cells.

12. Use of P1P (phytosphingosine-1-phosphate), cP1P (O-cyclic P1P), and/or NAPS-1-P (N-acetyl phytosphingosine-1-phosphate), or a derivative thereof for promoting osteogenic differentiation of adult stem cells.

13. A kit for promoting proliferation of adult stem cells *in vitro* comprising P1P (phytosphingosine-1-phosphate), cP1P (O-cyclic P1P), and/or NAPS-1-P (N-acetyl phytosphingosine-1-phosphate), or a derivative or a salt thereof.

14. A kit for inhibiting cell death of adult stem cells *in vitro* comprising P1P (phytosphingosine-1-phosphate), cP1P (O-cyclic P1P), and/or NAPS-1-P (N-acetyl phytosphingosine-1-phosphate), or a derivative or a salt thereof.

15. A kit for promoting osteogenic differentiation of adult stem cells *in vitro* comprising P1P (phytosphingosine-1-phosphate), cP1P (O-cyclic P1P), and/or NAPS-1-P (N-acetyl phytosphingosine-1-phosphate), or a derivative or a salt thereof.

16. A kit for culturing adult stem cells *in vitro* comprising P1P (phytosphingosine-1-phosphate), cP1P (O-cyclic P1P), and/or NAPS-1-P (N-acetyl phytosphingosine-1-phosphate), or a derivative or a salt thereof.
